# EUROPEAN PATENT APPLICATION

(11) **EP 4 273 228 A1**
(43) Date of publication of application: **08.11.2023**
(21) Application number: 21914495.3
(22) Date of filing: 29.12.2021
(51) Int. Cl.: C12N 1/21, C12N 15/31, C07K 14/34, C12P 13/14, C12R 1/15

(54) **STRAIN HAVING ENHANCED L-GLUTAMIC ACID PRODUCTIVITY, CONSTRUCTION METHOD THEREFOR AND APPLICATION THEREOF**

(30) Priority: 30.12.2020 CN 202011631311
(71) Applicant: Ningxia Eppen Biotech Co. Ltd, Ningxia 750100 (CN)
(72) Inventor: SU, Houbo, Yinchuan, Ningxia 750100 (CN); WEI, Aiying, Yinchuan, Ningxia 750100 (CN); MENG, Gang, Yinchuan, Ningxia 750100 (CN); YANG, Lipeng, Yinchuan, Ningxia 750100 (CN); MA, Fengyong, Yinchuan, Ningxia 750100 (CN); JIA, Huiping, Yinchuan, Ningxia 750100 (CN); ZHOU, Xiaoqun, Yinchuan, Ningxia 750100 (CN); ZHAO, Chunguang, Yinchuan, Ningxia 750100 (CN)
(74) Representative: ABG Intellectual Property Law, S.L.
(86) International application number: PCT/CN2021/142440
(87) International publication number: WO 2022/143763

(57) **Abstract**

Disclosed are strain having enhanced L-glutamic acid production capacity, and method for constructing the same and use thereof. A nucleotide sequence is provided by introducing a point mutation to a wild-type BBD29-00405 gene in *Corynebacterium glutamicum* so that the base at position 597 of SEQ ID NO: 1 is mutated from guanine (G) into adenine (A). Also provided is a recombinant strain obtained by introducing the polynucleotide sequence into L-glutamic acid-producing *Corynebacterium glutamicum,* the recombinant strain comprising a BBD29-00405 gene containing a point mutation. Compared with an unmodified strain, the resulting strain facilitates production of L-glutamic acid at a higher concentration.

## Description

### Technical Field

The present invention belongs to the technical field of gene engineering and microorganisms, and in particular relates to strain having enhanced L-glutamic acid production capacity, and method for constructing the same and use thereof.

### Background Art

L-glutamic acid has a chemical name: L-2-aminoglutaric acid; a molecular formula: C₅H₉O₄N; and a molecular weight: 147.13. L-glutamic acid is a nonessential amino acid. Glutamic acid existing in biological organisms belongs to L-type glutamic acid, and L-glutamic acid is a precursor of monosodium glutamate. After being eaten, monosodium glutamate can also be converted into glutamic acid in the stomach, then digested and absorbed to participate in the synthesis of protein, and other amino acids can be synthesized by transamination, which has a high value in nutrition. Apart from a wide range of applications in foods, it is also applied to medicine, cosmetics and agricultural production. Glutamate has the highest content in brain among the major organs of human body, and meanwhile glutamic acid is the only amino acid involved in the metabolism in human brain, which plays an important role in maintaining the function of nervous system. Therefore, for those suffering from neurasthenia, increasing glutamic acid intake can improve the function of nervous system. In cosmetic industry, glutamic acid can be used to synthesize sodium polyglutamate. Because of its strong hygroscopicity, it can be used as an emollient. Glutamic acid can also be combined with lauroyl chloride to generate sodium lauroyl glutamate, which is widely used in cosmetic industry. Glutamic acid mainly serves to produce bactericidal agents such as glutamate ketone in agriculture. In the process of fertilization, glutamic acid can also be used as a carrier of fertilizer to promote the absorption of nitrogen, phosphorus and potassium by crops.

In 1956, a glutamic acid-producing bacterium, *Corynebacterium glutamicum,* was isolated from nature, a major change in the history of monosodium glutamate production. In 1957, the era of producing monosodium glutamate by fermentation began. The success of glutamic acid production by fermentation is a great pioneering work of the whole fermentation industry, and it also greatly promotes the research and production of other fermented products. For industrial fermentation, the main factors that determine the level of fermentation production are strain performance, fermentation process, and downstream extraction process, etc. Among these factors, the acid production level of strains is the internal cause, a key to determine the success or failure of fermentation. Breeding of excellent strains is still the key to high-yield L-glutamic acid. With the progress in high-throughput screening technology, combined with traditional mutagenesis technology, it is helpful to find out high-yield L-glutamic acid mutant strains with unknown traits. These mutant strains may change the activity of a certain point in the metabolic pathway of L-glutamic acid or the recombination and integration along the whole pathway, so as to improve the acid production.

Although some strains that can increase the production of L-glutamic acid have been screened, more mutant strains for producing L-glutamic acid in high yield need to be found in order to meet the increasing demand.

### Summary

Aiming at the shortcomings of the prior art, the present invention provides a polynucleotide and a recombinant strain comprising the polynucleotide, and uses the recombinant strain to improve the L-glutamic acid production capacity of a bacterium. In order to achieve the above goals, the inventors of the present invention have found in research that the BBD29_00405 gene (GenBank: ANU32350.1) in the genome of *Corynebacterium glutamicum* ATCC13869 (GenBank: CP016335.1) having L-glutamic acid production capacity can be modified, or improved with respect to its expression to provide a recombinant strain with an increased production of L-glutamic acid. Compared with a wild-type strain that has not been modified, the capacity of the recombinant strain for producing L-glutamic acid is higher.

The present invention is implemented with the technical solutions as follows:
A first aspect of the present invention provides a bacterium for generating L-glutamic acid, having an improved expression of a polynucleotide encoding an amino acid sequence as set forth in SEQ ID NO: 3. According to the present invention, the improved expression is an enhanced expression of the polynucleotide, or having a point mutation in the polynucleotide encoding an amino acid sequence of SEQ ID NO: 3, or having a point mutation in, and an enhanced expression of the polynucleotide encoding an amino acid sequence of SEQ ID NO: 3.

The amino acid sequence of SEQ ID NO: 3 is a protein coded by the gene BBD29_00405.

The bacterium has an enhanced L-glutamic acid production capacity compared with an unmodified strain.

In the present invention, the term "bacterium having L-glutamic acid production capacity" refers to a bacterium having such a capacity of producing and accumulating target L-glutamic acid in a culture medium and/or in a cell of the bacterium that L-glutamic acid can be collected when the bacterium is cultured in the culture medium. A bacterium having L-glutamic acid production capacity can be a bacterium capable of accumulating target L-glutamic acid in a culture medium and/or in a cell of the bacterium in an amount greater than that with an unmodified strain.

The term "unmodified strain" refers to a control strain which has not been modified in a manner such that a specific feature is incorporated. That is, examples of the unmodified strain comprise a wild-type strain and a parent strain.

In the present invention, the term "L-glutamic acid" refers to L-glutamic acid in a free form, a salt thereof or a mixture thereof, unless otherwise specified.

The polynucleotide can encode an amino acid sequence having about 90% or more, about 92% or more, about 95% or more, about 97% or more, about 98% or more, or about 99% or more sequence homology with an amino acid sequence of SEQ ID NO: 3. As used herein, the term "homology" refers to percentage identity between two polynucleotides or two polypeptides as modules. Sequence homology can be measured between one module and another module using a method known in the art. For example, such sequence homology can be measured by virtue of BLAST algorithm.

The expression of a polynucleotide can be enhanced: by substituting or mutating an expression regulatory sequence, introducing a mutation to the sequence of the polynucleotide, and by increasing the copy number of the polynucleotide inserted via a chromosome or introduced with a vector, or a combination thereof, etc.

The expression regulatory sequence of a polynucleotide can be modified. The expression regulatory sequence controls the expression of a polynucleotide operably linked thereto, and can comprise, for example, promoters, terminators, enhancers, silencers and the like. A polynucleotide can have a change in an initiation codon. A polynucleotide can be incorporated into a specific site of a chromosome, thereby increasing copy number. Herein, a specific site can comprise, for example, a transposon site or an intergenic site. In addition, a polynucleotide can be incorporated into an expression vector, and the expression vector is introduced into a host cell, thus increasing copy number.

In an embodiment of the present invention, a polynucleotide, or a polynucleotide having a point mutation is incorporated into a specific site of a chromosome of a microorganism, thus increasing copy number.

In an embodiment of the present invention, a polynucleotide with a promoter sequence, or a polynucleotide having a point mutation with a promoter sequence is incorporated into a specific site of a chromosome of a microorganism, thus overexpressing the nucleic sequence.

In an embodiment of the present invention, a polynucleotide, or a polynucleotide having a point mutation is incorporated into an expression vector, and the expression vector is introduced into a host cell, thus increasing copy number.

In an embodiment of the present invention, a polynucleotide with a promoter sequence, or a polynucleotide having a point mutation with a promoter sequence is incorporated into an expression vector, and the expression vector is introduced into a host cell, thus overexpressing the amino acid sequence.

In a specific embodiment of the present invention, the polynucleotide can comprise a nucleotide sequence of SEQ ID NO: 1.

In an embodiment of the present invention, the polynucleotide encoding an amino acid sequence of SEQ ID NO: 3 has a point mutation such that methionine at position 199 in the amino acid sequence of SEQ ID NO: 3 is substituted with a different amino acid.

According to the present invention, preferably methionine at position 199 is substituted with isoleucine.

According to the present invention, as set forth in SEQ ID NO: 4 is the amino acid sequence following substitution with isoleucine of methionine at position 199 in an amino acid sequence set forth in SEQ ID NO: 3.

In an embodiment of the present invention, the polynucleotide sequence having a point mutation is formed from a mutation to the base at position 597 of a polynucleotide sequence set forth in SEQ ID NO: 1.

According to the present invention, the mutation comprises a mutation of the base at position 597 of a polynucleotide sequence set forth in SEQ ID NO: 1 from guanine (G) to adenine (A).

In an embodiment of the present invention, the polynucleotide sequence having a point mutation comprises a polynucleotide sequence set forth in SEQ ID NO: 2.

As used herein, the term "operably linked" refers to a functional link between a regulatory sequence and a polynucleotide sequence, whereby the regulatory sequence controls transcription and/or translation of the polynucleotide sequence. A regulatory sequence can be a strong promoter which can enhance the expression level of a polynucleotide. A regulatory sequence can be a promoter derived from a microorganism belonging to the genus *Corynebacterium* or can be a promoter derived from other microorganisms. For example, the promoter can be trc promoter, gap promoter, tac promoter, T7 promoter, lac promoter, trp promoter, araBAD promoter or cj7 promoter.

In a specific embodiment of the present invention, the promoter is a promoter of a polynucleotide encoding an amino acid sequence of SEQ ID NO: 3 (BBD29_00405).

As used herein, the term "vector" refers to a polynucleotide construct containing a regulatory sequence of a gene and a gene sequence and configured to express a target gene in a suitable host cell. Or, a vector can also refer to a polynucleotide construct containing a sequence for homologous recombination such that due to the vector introduced into a host cell, the regulatory sequence of an endogenous gene in the genome of the host cell can be changed, or a target gene that can be expressed can be inserted into a specific site of the genome of a host. In this regard, the vector used in the present invention can further comprise a selective marker to determine the introduction of the vector into a host cell or the insertion of the vector into the chromosome of a host cell. A selective marker can comprise a marker providing a selectable phenotype, such as drug resistance, auxotroph, resistance to cytotoxic agents, or expression of a surface protein. In an environment treated with such a selective agent, the transformed cell can be selected since only the cell that expresses the selective marker can survive or display different phenotypic traits. The vector as described herein is well known to those skilled in the art, including but not limited to: plasmid, bacteriophage (such as λ bacteriophage or M13 filamentous bacteriophage etc.), cosmid (i.e., Cosmid) or viral vector.

In some specific embodiments of the present invention, the vector used is pK18mobsacB plasmid, and pXMJ19 plasmid.

As used herein, the term "transformation" refers to introduction of a polynucleotide into a host cell, such that the polynucleotide can be replication-competent as an element foreign to a genome or by being inserted into the genome of a host cell. A method for transforming the vector used in the present invention can comprise a method for introducing a nucleic acid into a cell. In addition, as disclosed in the related techniques, a method with electric pulse can be performed according to a host cell.

Herein, the microorganism can be yeast, bacterium, alga or fungi.

According to the present invention, the bacterium can be a microorganism belonging to the genus *Corynebacterium,* such as *Corynebacterium acetoacidophilum, Corynebacterium acetoglutamicum, Corynebacterium callunae, Corynebacterium glutamicum, Brevibacterium flavum, Brevibacterium lactofermentum, Corynebacterium ammoniagenes, Corynebacterium pekinense, Brevibacterium saccharolyticum, Brevibacterium roseum,* and *Brevibacterium thiogenitalis,* etc.

In an implementation of the present invention, the microorganism belonging to the genus *Corynebacterium* is *Corynebacterium glutamicum* ATCC13869.

In an implementation of the present invention, the microorganism belonging to the genus *Corynebacterium* is *Corynebacterium glutamicum* YPGLU001, and has been deposited at China General Microbiological Culture Collection Center, abbreviated as CGMCC, Address: No. 3, Yard 1, Beichen West Road, Chaoyang District, Beijing, Post Code: 100101 on November 23, 2020, with Accession Number of the Biological Deposit: CGMCC No. 21220.

A second aspect of the present invention provides a polynucleotide sequence, an amino acid sequence coded by the polynucleotide sequence, a recombinant vector comprising the polynucleotide sequence, and a recombinant strain containing the polynucleotide sequence.

According to the present invention, the polynucleotide sequence comprises a polynucleotide encoding a polypeptide containing an amino acid sequence set forth in SEQ ID NO: 3 with methionine at position 199 in the sequence substituted with a different amino acid.

According to the present invention, preferably methionine at position 199 is substituted with isoleucine.

According to the present invention, as set forth in SEQ ID NO: 4 is the amino acid sequence following substitution with isoleucine of methionine at position 199 in an amino acid sequence set forth in SEQ ID NO: 3.

According to the present invention, preferably the polynucleotide sequence encoding a polypeptide containing an amino acid sequence set forth in SEQ ID NO: 3 contains a polynucleotide sequence as set forth in SEQ ID NO: 1.

In an embodiment of the present invention, the polynucleotide sequence is formed from a mutation to the base at position 597 in a polynucleotide sequence set forth in SEQ ID NO: 1.

According to the present invention, the mutation refers to a change in the base/nucleotide of the site, and the method for mutation can be at least one selected from mutagenesis, PCR site-directed mutation, and/or homologous recombination, etc. In the present invention, PCR site-directed mutation and/or homologous recombination are preferably used.

According to the present invention, the mutation comprises a mutation at position 597 in a polynucleotide sequence set forth in SEQ ID NO: 1 from guanine (G) to adenine (A).

In an embodiment of the present invention, the polynucleotide sequence comprises a polynucleotide sequence set forth in SEQ ID NO: 2.

According to the present invention, the amino acid sequence comprises an amino acid sequence set forth in SEQ ID NO: 4.

According to the present invention, the recombinant vector is constructed by introducing the polynucleotide sequence to a plasmid.

In an embodiment of the present invention, the plasmid is pK18mobsacB plasmid.

In another embodiment of the present invention, the plasmid is pXMJ19 plasmid.

Specifically, the polynucleotide sequence and the plasmid can be constructed as a recombinant vector by virtue of NEBuider recombination system.

According to the present invention, the recombinant strain contains the polynucleotide sequence.

As an implementation of the present invention, a starting strain of the recombinant strain is *Corynebacterium glutamicum* YPGLU001 with Accession Number of the Biological Deposit: CGMCC No. 21220.

As an implementation of the present invention, a starting strain of the recombinant strain is ATCC 13869.

A third aspect of the present invention provides use of the polynucleotide sequence, an amino acid sequence coded by the polynucleotide sequence, a recombinant vector comprising the polynucleotide sequence, and a recombinant strain containing the polynucleotide sequence in the production of L-glutamic acid.

A fourth aspect of the present invention further provides a method for constructing a recombinant strain generating L-glutamic acid.

According to the present invention, the method for constructing comprises the step of:
engineering a polynucleotide sequence of a wild-type BBD29_00405 gene as set forth in SEQ ID NO: 1 in a host strain to cause a mutation to the base at position 597 to provide a recombinant strain containing a mutated BBD29_00405 coding gene.

According to the method for constructing of the present invention, the engineering comprises at least one of mutagenesis, PCR site-directed mutation, and/or homologous recombination, etc.

According to the method for constructing of the present invention, the mutation refers to a change of the base at position 597 in SEQ ID NO: 1 from guanine (G) to adenine (A); specifically, the polynucleotide sequence containing a mutated BBD29_00405 coding gene is set forth in SEQ ID NO: 2.

Further, the method for constructing comprises the steps of:
(1) engineering a nucleotide sequence of a wild-type BBD29_00405 gene as set forth in SEQ ID NO: 1 to cause a mutation to the base at position 597 to provide a mutated BBD29_00405 gene polynucleotide sequence;
(2) linking the mutated polynucleotide sequence to a plasmid to construct a recombinant vector;
(3) introducing the recombinant vector into a host strain to provide a recombinant strain containing a mutated BBD29_00405 coding gene.

According to the method for constructing of the present invention, the step (1) comprises constructing a BBD29_00405 gene with a point mutation: according to a genome sequence of an unmodified strain, synthesizing two pairs of primers P1, P2 and P3, P4 for amplifying a fragment of the BBD29_00405 gene, and introducing a point mutation to a wild-type BBD29_00405 gene, SEQ ID NO: 1, by virtue of PCR site-directed mutation to provide a BBD29_00405 gene nucleotide sequence with a point mutation, SEQ ID NO: 2, designated as BBD29_00405^{G597A}.

In an embodiment of the present invention, the genome of the unmodified strain can be derived from the strain ATCC13869, and its genome sequence GenBank: CP016335.1 can be available from the NCBI website.

In an implementation of the present invention, in the step (1), the primers are:
P1: 5'CAGTGCCAAGCTTGCATGCCTGCAGGTCGACTCTAGATGACTATTAATGTC TCCGA 3' (SEQ ID NO: 5)
P2: 5'AGACCGGCATCAAGTATGGTCTGGGCA3' (SEQ ID NO: 6)
P3: 5'TGCCCAGACCATACTTGATGCCGGTCT3' (SEQ ID NO: 7)
P4: 5'CAGCTATGACCATGATTACGAATTCGAGCTCGGTACCCCTAGCCGGCGTAA GGATCCCGGAT3' (SEQ ID NO: 8)

In an implementation of the present invention, the PCR amplification is performed in the following manner: pre-denaturation for 5 min at 94°C, denaturation for 30 s at 94°C, annealing for 30 s at 52°C, and extension for 40 s at 72°C (30 circles), and overextension for 10 min at 72°C.

In an implementation of the present invention, the overlap PCR amplification is performed in the following manner: pre-denaturation for 5 min at 94°C, denaturation for 30 s at 94°C, annealing for 30 s at 52°C, and extension for 100 s at 72°C (30 circles), and overextension for 10 min at 72°C.

According to the method for constructing of the present invention, the step (2) comprises constructing a recombinant plasmid, comprising assembling an isolated and purified BBD29_00405^{G597A} with pK18mobsacB plasmid by virtue of NEBuider recombination system to provide a recombinant plasmid.

According to the method for constructing of the present invention, the step (3) comprises constructing a recombinant strain by transforming a recombinant plasmid to a host strain to provide a recombinant strain.

In an implementation of the present invention, the transforming in the step (3) is by an electrotransformation method.

In an embodiment of the present invention, the host strain is ATCC 13869.

In an embodiment of the present invention, the host strain is *Corynebacterium glutamicum* YPGLU001 with Accession Number of the Biological Deposit: CGMCC No. 21220.

In an embodiment of the present invention, the recombination is achieved by homologous recombination.

A fifth aspect of the present invention further provides a method for constructing a recombinant strain generating L-glutamic acid.

According to the present invention, the method for constructing comprises the step of:
amplifying upstream and downstream homology arm fragments of BBD29_00405 and a sequence of a BBD29_00405 gene coding region and a promoter region thereof, and introducing BBD29_00405 or BBD29_00405^{G597A} gene into the genome of a host strain in a manner of homologous recombination so as to enable the strain to overexpress the BBD29_00405 or BBD29_00405^{G597A}gene.

In an embodiment of the present invention, the primers for amplifying the upstream homology arm fragments are:
P7: 5'CAGTGCCAAGCTTGCATGCCTGCAGGTCGACTCTAGGACCCGCTTGCCAT ACGAAG 3'
P8: 5'CCTACCACGA CGAGCACTAC ATCTACTCAT CTGAAGAATC 3'

In an embodiment of the present invention, the primers for amplifying the downstream homologous arm fragments are:
P11: 5'GGGATCCTTA CGCCGGCTAG TTCGTGGGCA CTCTGGTTTG 3'
P12:5'CAGCTATGACCATGATTACGAATTCGAGCTCGGTACCCCATAAGAAACA ACCACTTCC3'

In an embodiment of the present invention, the primers for amplifying the sequence of the gene coding region and the promoter region thereof are:
P9: 5'GATTCTTCAG ATGAGTAGAT GTAGTGCTCG TCGTGGTAGG 3'(SEQ ID NO:13)
P10: 5'CAAACCAGAG TGCCCACGAA CTAGCCGGCG TAAGGATCCC 3'(SEQ ID NO: 14)

In an embodiment of the present invention, with the above-mentioned P7/P12 as primers again, amplification is performed using as templates a mixture of three fragments, the amplified upstream homology arm fragment, downstream homology arm fragment, and fragment of the sequence of the gene coding region and the promoter region thereof, to provide an integrated homology arm fragment.

In an embodiment of the present invention, the PCR system used comprises: 10 × Ex Taq Buffer 5 µL, dNTP Mixture (2.5 mM each) 4 µL, Mg²⁺ (25 mM) 4 µL, primers (10 pM) 2 µL each, and Ex Taq (5 U/µL) 0.25 µL in a total volume of 50 µL; the PCR amplification is performed in the following manner: pre-denaturation for 5 min at 94°C, denaturation for 30 s at 94°C, annealing for 30 s at 52°C, and extension for 180 s at 72°C (30 circles), and overextension for 10 min at 72°C.

In an embodiment of the present invention, NEBuider recombination system is used to assemble shuttle plasmid PK18mobsacB with the integrated homology arm fragment to provide an integrative plasmid.

In an embodiment of the present invention, a host strain is transfected with the integrative plasmid to introduce BBD29_00405 or BBD29_00405^{G597A} gene into the genome of the host strain in a manner of homologous recombination.

In an embodiment of the present invention, the host strain is *Corynebacterium glutamicum* YPGLU001 with Accession Number of the Biological Deposit: CGMCC No. 21220.

In an embodiment of the present invention, the host strain is ATCC 13869.

In an embodiment of the present invention, the host strain is a strain bearing a polynucleotide sequence set forth in SEQ ID NO: 2.

A sixth aspect of the present invention further provides a method for constructing a recombinant strain producing L-glutamic acid.

According to the present invention, the method for constructing comprises the steps of:
amplifying the sequence of BBD29_00405 gene coding region and a promoter region, or the sequence of BBD29_00405^{G597A} gene coding region and a promoter region, constructing an overexpression plasmid vector, and transforming the vector into a host strain to enable the strain to overexpress BBD29_00405 or BBD29_00405 ^{G597A} gene.

In an embodiment of the present invention, the primers for amplifying the sequence of the gene coding region and the promoter region thereof are:
P17: 5'GCTTGCATGCCTGCAGGTCGACTCTAGAGGATCCCC GTAG TGCTCG TCGTGGTAGG 3' (SEQ ID NO: 21)
P18: 5'ATCAGGCTGAAAATCTTCTCTCATCCGCCAAAACCTAGCCGGCG TAAGGATCCCGGAT 3' (SEQ ID NO: 22)

In an embodiment of the present invention, the PCR system comprises: 10 × Ex Taq Buffer 5 µL, dNTP Mixture (2.5 mM each) 4 µL, Mg²⁺ (25 mM) 4 µL, primers (10 pM) 2 µL each, Ex Taq (5 U/µL) 0.25 µL, in a total volume of 50 µL. The PCR amplification is performed in the following manner: pre-denaturation for 5 min at 94°C, denaturation for 30 s at 94°C, annealing for 30 s at 52°C, extension for 100 s at 72°C (30 circles), and overextension for 10 min at 72°C.

In an embodiment of the present invention, NEBuider recombination system is used to assemble shuttle plasmid pXMJ19 with BBD29 00405 or BBD29_00405^{G597A} fragment having its own promoters to provide an overexpression plasmid.

In an embodiment of the present invention, the host strain is *Corynebacterium glutamicum* YPGLU001 with Accession Number of the Biological Deposit: CGMCC No. 21220.

In an embodiment of the present invention, the host strain is ATCC 13869.

In an embodiment of the present invention, the host strain is a strain bearing a polynucleotide sequence set forth in SEQ ID NO: 2.

The recombinant strain provided in the present invention can be individually applied to fermentation for producing L-glutamic acid, and can also be in hybrid fermentation with other L-glutamic acid-producing bacteria for producing L-glutamic acid.

Another aspect of the present invention provides a method for producing L-glutamic acid, the method comprising culturing the bacterium; and obtaining L-glutamic acid from a culture.

A bacterium can be cultured in a suitable culture medium under culturing conditions as known in the art. The culture medium can comprise: carbon source, nitrogen source, trace elements, and a combination thereof. In culturing, pH of the culture can be adjusted. In addition, bubbles can be prevented from generating in culturing, for example, by using a defoamer to prevent bubbles generation. Further, gases can be injected into the culture in culturing. The gases can comprise any ones capable of maintaining an aerobic condition for the culture. In culturing, the temperature of the culture can be from 20°C to 45°C. The generated L-glutamic acid can be recovered from the culture, i.e., the culture is treated with sulfuric acid or hydrochloric acid, etc. followed by a combination of methods such as anion-exchange chromatography, condensation, crystallization, and isoelectric precipitation.

The present invention also provides a protein, designated as protein BBD29_00405^{M199I}, wherein the protein can be any one of:
A1) a protein whose amino acid sequence is SEQ ID NO: 4;
A2) a protein having 80% or more identity to and the same function as the protein indicated in A1), as obtained by subjecting the amino acid sequence set forth in SEQ ID NO: 4 to substitution and/or deletion and/or addition of amino acid residues;
A3) a fusion protein having the same function, as obtained by linking a tag to the N-terminus and/or C-terminus of A1) or A2).

The present invention also provides a nucleic acid molecule, designated as BBD29_00405^{G597A}, wherein the nucleic acid molecule BBD29_00405^{G597A} can be any one of:
B1) a nucleic acid molecule encoding the protein BBD29_00405^{M199I};
B2) a DNA molecule whose coding sequence is set forth in SEQ ID NO: 2;
B3) a DNA molecule whose nucleotide sequence is set forth in SEQ ID NO: 2.

The DNA molecule set forth in SEQ ID NO: 2 is the BBD29_00405^{G597A} gene of the present invention.

The DNA molecule set forth in SEQ ID NO: 2 (the BBD29_00405^{G597A} gene) encodes the protein BBD29_00405^{M199I} set forth in SEQ ID NO: 4.

The amino acid sequence of the protein BBD29_00405^{M199I} (SEQ ID NO: 4) is derived from **a change of methionine (M)** at position 199 of **SEQ ID NO: 3 to isoleucine (I).**

The present invention also provides a biomaterial, wherein the biomaterial can be any one of:
C1) an expression cassette comprising the nucleic acid molecule BBD29_00405^{G597A};
C2) a recombinant vector comprising the nucleic acid molecule BBD29_00405^{G597A}, or a recombinant vector comprising the expression cassette of C1);
C3) a recombinant microorganism comprising the nucleic acid molecule BBD29_00405^{G597A}, or a recombinant microorganism comprising the expression cassette of C1), or a recombinant microorganism comprising the recombinant vector of C2).

The present invention also provides any one of the following uses of any one of D1)-D8):
F1) use of any one of D1)-D8) in the regulation of the production of L-glutamic acid of a microorganism;
F2) use of any one of D1)-D8) in the construction of a genetically engineered bacterium for producing L-glutamic acid;
F3) use of any one of D1)-D8) in the preparation of L-glutamic acid;
wherein the D1)-D8) is:
D1) the protein BBD29_00405^{M199I};
D2) the nucleic acid molecule BBD29_00405^{G597A};
D3) the biomaterial;
D4) a DNA molecule whose nucleotide sequence is SEQ ID NO: 1;
D5) a DNA molecule having 90% or more identity to and the same function as the DNA molecule set forth in SEQ ID NO: 1, as obtained by subjecting the nucleotide sequence set forth in SEQ ID NO: 1 to modification, and/or substitution and/or deletion and/or addition of one or several nucleotides;
D6) an expression cassette comprising the DNA molecule in D4) or D5);
D7) a recombinant vector comprising the DNA molecule in D4) or D5), or a recombinant vector comprising the expression cassette of D6);
D8) a recombinant microorganism comprising the DNA molecule in D4) or D5), or a recombinant microorganism comprising the expression cassette of D6), or a recombinant microorganism comprising the recombinant vector of D7).

The DNA molecule set forth in SEQ ID NO: 1 is the BBD29_00405 gene of the present invention.

The DNA molecule set forth in SEQ ID NO: 1 (the BBD29_00405 gene) encodes a protein set forth in SEQ ID NO: 3.

Herein, identity refers to the identity between amino acid sequences or nucleotide sequences. International homology retrieval sites on the Internet can be employed to determine the identity between amino acid sequences, such as the BLAST page on the NCBI homepage website. For example, in Advanced BLAST2.1, blastp can be used as a program, with the Expect value set at 10, each Filter set to OFF, BLOSUM62 used as Matrix, and Gap existence cost, Per residue gap cost and Lambda ratio set at 11, 1 and 0.85 (default values), respectively, and a search is done for the identity between a pair of amino acid sequences for calculation, and then a value of identity (%) can be available.

Herein, the 80% or more identity can be an identity of at least 80%, 81%, 82%, 83%, 84%, 85%, 86%, 87%, 88%, 89%, 90%, 91%, 92%, 93%, 94%, 95%, 96%, 97%, 98% or 99%.

Herein, the 90% or more identity can be an identity of at least 90%, 91%, 92%, 93%, 94%, 95%, 96%, 97%, 98% or 99%.

The regulation of the production of L-glutamic acid of a microorganism as described herein can be increasing or reducing the amount of the accumulation of L-glutamic acid in the microorganism (i.e., promoting or inhibiting the biosynthesis of L-glutamic acid).

The present invention also provides a method for increasing the production of L-glutamic acid in a microorganism, the method comprising any one of:
E1) increasing the expression amount, or content of the nucleic acid molecule BBD29_00405^{G597A} in a target microorganism to provide a microorganism having a greater production of L-glutamic acid than the target microorganism;
E2) increasing the expression amount, or content of the DNA molecule of D4) or D5) in a target microorganism to provide a microorganism having a greater production of L-glutamic acid than the target microorganism;
E3) performing a mutation on the DNA molecule whose nucleotide sequence is SEQ ID NO: 1 in the target microorganism to provide a microorganism having a greater production of L-glutamic acid than the target microorganism.

In the method above, the mutation can be a point mutation, i.e., a mutation of a single nucleotide.

In the method above, the point mutation can be a mutation of alanine residue at position 199 in an amino acid sequence coded by the DNA molecule set forth in SEQ ID NO: 1 to another amino acid residue.

In the method above, the point mutation can be a mutation of alanine at position 199 in an amino acid sequence coded by the DNA molecule set forth in SEQ ID NO: 1 to threonine, providing a mutated protein BBD29_00405^{M199I} whose amino acid sequence is SEQ ID NO: 4.

The mutation refers to a change in one or several bases in a gene through site-directed mutation, resulting in a change in the constitution of the amino acids of a corresponding protein, thereby generating a new protein or causing a new function to the original protein, that is, site-directed mutation to genes. Techniques for site-directed mutation to genes such as oligonucleotide primer-mediated site-directed mutation, PCR-mediated site-directed mutation, or cassette mutation are well known to those skilled in the art.

The point mutation as described herein can be replacement of a single base, insertion of a single base, or deletion of a single base, and specifically, replacement of a single base. The replacement of a single base can be allelic replacement.

The point mutation can be performing a nucleic acid modification to guanine (G) at position 597 of the BBD29_00405 gene (SEQ ID NO: 1).

Specifically, the point mutation can be performing a mutation to guanine (G) at position 597 of the BBD29_00405 gene (SEQ ID NO: 1) to adenine (A) to provide a DNA molecule set forth in SEQ ID NO: 2.

Herein, the recombinant vector can be, specifically, recombinant vector pK18-BBD29_00405^{G597A}, PK18mobsacB-BBD29_00405, PK18mobsacB-BBD29_00405^{G597A}, pXMJ19-BBD29_00405 or pXMJ19-BBD29_00405^{G597A};

The recombinant vector pK18-BBD29_00405^{G597A} contains the DNA molecule set forth as positions 1-1473 of the mutated gene BBD29_00405^{G597A} set forth in SEQ ID NO: 2, and specifically is a recombinant vector obtained by inserting the DNA fragment BBD29_00405^{G597A}-Up-Down fragment set forth in SEQ ID NO: 31 between *Xbα* I recognition sites in pK18mobsacB vector, while keeping the other sequences of pK18mobsacB vector unchanged.

The recombinant vector PK18mobsacB-BBD29_00405 is used to integrate an exogenous gene BBD29_00405 into a chromosome of a host, and to overexpress a wild-type BBD29_00405 gene in a producer bacterium.

The recombinant vector PK18mobsacB-BBD29_00405^{G597A} is used to integrate an exogenous gene BBD29_00405^{G597A} to a chromosome of a host, and to overexpress a mutant gene BBD29_00405^{G597A} in a producer bacterium.

The recombinant vector pXMJ19-BBD29_00405 is used to express an exogenous gene BBD29_00405 outside a chromosome via a plasmid, thereby overexpressing a wild-type BBD29_00405 gene in a producer bacterium.

The recombinant vector pXMJ19-BBD29_00405^{G597A} is used to express an exogenous gene BBD29_00405^{G597A} outside a chromosome via a plasmid, thereby overexpressing a mutant gene BBD29_00405^{G597A} in a producer bacterium.

The recombinant vectors pK18-BBD29_00405^{G597A}, PK18mobsacB-BBD29_00405, PK18mobsacB-BBD29_00405^{G597A}, pXMJ19-BBD29_00405 and pXMJ19-BBD29_00405^{G597A} are all within the scope of protection of the present invention.

Herein, the recombinant microorganism can be, specifically, a recombinant bacterium YPG-025, YPG-026, YPG-027, YPG-028 or YPG-029.

The recombinant bacterium YPG-025 is a recombinant bacterium obtained by transformation of the recombinant vector pK18-BBD29_00405^{G597A} into *Corynebacterium glutamicum* CGMCC No. 21220, and the recombinant bacterium YPG-025 contains the mutated gene BBD29_00405^{G597A} set forth in SEQ ID NO: 2.

The recombinant bacterium YPG-026 contains a double-copy BBD29_00405 gene set forth in SEQ ID NO: 1; a recombinant bacterium containing a double-copy BBD29_00405 gene can significantly and steadily increase the expression amount of BBD29_00405 gene. The recombinant bacterium YPG-026 is an engineered bacterium overexpressing a wild-type BBD29_00405 gene on genome.

The recombinant bacterium YPG-027 contains a mutated BBD29_00405^{G597A} gene set forth in SEQ ID NO: 2; the recombinant bacterium YPG-027 is an engineered bacterium overexpressing a mutant-type BBD29_00405^{G597A} gene on genome.

The recombinant bacterium YPG-028 contains a double-copy BBD29_00405 gene set forth in SEQ ID NO: 1; the recombinant bacterium YPG-028 is an engineered bacterium overexpressing a wild-type BBD29_00405 gene on a plasmid, i.e., overexpression outside a chromosome via a plasmid pXMJ19-BBD29_00405.

The recombinant bacterium YPG-029 contains a mutated BBD29_00405^{G597A} gene set forth in SEQ ID NO: 2; the recombinant bacterium YPG-029 is an engineered bacterium overexpressing a mutant-type BBD29_00405^{G597A} gene on a plasmid, i.e., overexpression outside a chromosome via a plasmid pXMJ19-BBD29_00405^{G597A};

The recombinant bacteria YPG-025, YPG-025, YPG-027, YPG-027 and YPG-029 are all within the scope of protection of the present invention.

The present invention also provides a method for constructing the recombinant microorganism, the method comprising at least one of:
F1) introducing the nucleic acid molecule BBD29_00405^{G597A} into a target microorganism to provide the recombinant microorganism;
F2) introducing the DNA molecule set forth in SEQ ID NO: 1 into a target microorganism to provide the recombinant microorganism;
F3) editing the DNA molecule set forth in SEQ ID NO: 1 with a gene editing measure (such as single-base gene editing) to contain the DNA molecule set forth in SEQ ID NO: 2 in a target microorganism.

The introducing can be host bacterium transformation with a vector bearing a DNA molecule of the present invention through any of known transformation methods, such as a chemical transformation method or an electrotransformation method. The introduced DNA molecule can be single-copy, or multi-copy. The introducing can be integration of an exogenous gene into a chromosome of a host, or expression outside a chromosome via a plasmid.

The present invention also provides a method for preparing L-glutamic acid, the method comprising producing L-glutamic acid with any one of the recombinant microorganisms as described herein.

In the method above, the method can be preparation of L-glutamic acid in a fermentation method, and the recombinant microorganism can be the genus *Corynebacterium,* and specifically, *Corynebacterium glutamicum* and variants thereof.

### Reference to Biological Deposit

*Corynebacterium glutamicum* was deposited at China General Microbiological Culture Collection Center, abbreviated as CGMCC, Address: No. 3, Yard 1, Beichen West Road, Chaoyang District, Beijing, Post Code: 100101 on November 23, 2020, with Accession Number of the Biological Deposit: CGMCC No. 21220, Strain Name: YPGLU001.

### Best Modes for Carrying out the Invention

In the following, the technical solutions of the present invention will be further described in detail in connection with specific examples. It should be understood that the following examples merely illustrate and explain the present invention, and should not be construed as limiting the scope of protection of the present invention. All the techniques implemented based on the above contents of the present invention are encompassed in the scope that the present invention intends to protect.

Unless otherwise specified, the raw materials and reagents used in the following examples are all commercially available or can be prepared by known methods. The experimental methods without specific conditions annotated in the following examples usually adhere to routine conditions such as those described in Sambrook et al., Molecular Cloning: Laboratory Manual (New York: Cold Spring Harbor Laboratory Press, 1989), or to those recommended by manufacturers.

Unless otherwise defined or expressly indicated by the background, all the technical and scientific terms in the disclosure have the same meaning as commonly understood by one of ordinary skilled in the art to which the disclosure pertains.

In the following examples, the constitution of the basic medium used to culture the strain is the same, and sucrose, kanamycin or chloramphenicol, etc. are added based on the constitution of the basic medium for a corresponding demand. If a solid one is desired, just add 2% agar, with pH at 7.0 and temperature at 30°C for culturing. The constitution of solutes in a basic medium is shown in Table 1 below. Dissolve the solutes shown in Table 1 in water to provide a basic medium:

**Table 1 Constitution of Basic Medium**

| Name of Reagent | Compounding Ratio | Fermentation Tank |
|---|---|---|
| Glucose | g/L | 5.0 |
| Phosphoric Acid | g/L | 0.38 |
| Magnesium Sulfate | g/L | 1.85 |
| Potassium Chloride | g/L | 1.6 |
| Biotin | µg/L | 550 |
| Vitamin B1 | µg/L | 300 |
| Ferrous Sulfate | mg/L | 10 |
| Manganese Sulfate | g/dl | 10 |
| KH₂PO₄ | g/L | 2.8 |
| Vitamin C | mg/L | 0.75 |
| Vitamin B12 | µg/L | 2.5 |
| Para-Aminobenzoic acid | mg/L | 0.75 |
| Defoamer | ml/dl | 0.0015 |
| Betaine | g/L | 1.5 |
| Cane-Sugar Molasses | ml/L | 7 |
| Corn Steep Liquor | ml/L | 77 |
| Aspartic acid | g/L | 1.7 |
| Hair powder | g/L | 2 |

*Corynebacterium glutamicum* YPGLU001 CGMCC No. 21220 in the following examples has been deposited at China General Microbiological Culture Collection Center (abbreviated as CGMCC, Address: No. 3, Yard 1, Beichen West Road, Chaoyang District, Beijing, Institute of Microbiology, Chinese Academy of Sciences) on November 23, 2020, with Accession No. CGMCC No. 21220. *Corynebacterium glutamicum* YPGLU001 is also known as *Corynebacterium glutamicum* CGMCC No. 21220.

### Example 1. Construction of Transformation Vector pK18-BBD29_00405^{G597A} Comprising BBD29_00405 Gene Coding Region with Point Mutation

According to the *Corynebacterium glutamicum* ATCC13869 genome (GenBank: CP016335.1) sequence published on NCBI, two pairs of primers are designed and synthesized for amplifying a sequence of a BBD29_00405 gene (GenBank: AKF27993.1) coding zone to introduce a point mutation to the strains ATCC13869 and *Corynebacterium glutamicum* YPGLU001 (Accession Number of the Biological Deposit: CGMCC No. 21220) producing L-glutamic acid in high yield by virtue of allelic replacement. The amino acid sequence corresponding to a coded protein is SEQ ID NO: 3, with a change of guanine (G) at position 597 in the nucleotide sequence of BBD29_00405 gene to adenine (A) (SEQ ID NO: 2: BBD29_00405^{G597A}), and thus a change of methionine (M) at position 199 in the amino acid sequence corresponding to the coded protein to isoleucine (I) (SEQ ID NO: 4: BBD29_00405^{M199I}).

The nucleotide sequence of a wild-type BBD29_00405 gene is SEQ ID NO: 1 and the amino acid sequence of the BBD29_00405 protein coded by it is SEQ ID NO: 3;

The nucleotide sequence of a mutated BBD29_00405^{G597A} gene is SEQ ID NO: 2 and the amino acid sequence of the mutated BBD29_00405^{M199I} protein coded by it is SEQ ID NO: 4.

The primers are designed as follows (synthesized by Invitrogen Corporation, Shanghai, with mutated base underlined):
P1: 5' CAGTGCCAAGCTTGCATGCCTGCAGGTCGACTCTAGATGACTATTAATGTCTCCGA 3' (SEQ ID NO: 5)
P2: 5'AGACCGGCATCAAGTATGGTCTGGGCA3'(SEQ ID NO: 6)
P3: 5'TGCCCAGACCATACTTGATGCCGGTCT3'(SEQ ID NO: 7)
P4: 5'CAGCTATGACCATGATTACGAATTCGAGCTCGGTACCCCTAGCCGGCGTAA GGATCCCGGAT 3'(SEQ ID NO: 8)

Method for Construction: using *Corynebacterium glutamicum* ATCC13869 as a template, PCR amplification is performed with respective primers P1, P2 and P3, P4.

PCR System: 10 × Ex Taq Buffer 5 µL, dNTP Mixture (2.5 mM each) 4 µL, Mg²⁺ (25 mM) 4 µL, primers (10 pM) 2 µL each, Ex Taq (5 U/µL) 0.25 µL, a template 1 µL, with water as balance, in a total volume of 50 µL.

The above PCR amplification is performed by pre-denaturation for 5 min at 94°C, denaturation for 30 s at 94°C, annealing for 30 s at 52°C, and extension for 40 s at 72°C (30 circles), and overextension for 10 min at 72°C, to provide two DNA fragments containing a BBD29_00405 gene coding region each sized in about 647 bp and 927 bp, respectively, (BBD29_00405^{G597A}-Up (SEQ ID NO: 29) and BBD29_00405^{G597A}-Down (SEQ ID NO: 30).

After BBD29_00405^{G597A}-Up and BBD29_00405^{G597A}-Down are isolated via Agarose Gel Electrophoresis and purified, the above two DNA fragments are then used as a template with P1 and P4 as primers for overlap PCR amplification to provide a BBD29_00405^{G597A}-Up-Down fragment in 1547 bp in length, having a nucleotide sequence of SEQ ID NO: 31.

PCR System: 10 × Ex Taq Buffer 5 µL, dNTP Mixture (2.5 mM each) 4 µL, Mg²⁺ (25 mM) 4 µL, primers (10 pM) 2 µL each, Ex Taq (5 U/µL) 0.25 µL, a template 1 µL, with water as balance, in a total volume of 50 µL.

The above PCR amplification is performed by pre-denaturation for 5 min at 94°C, denaturation for 30 s at 94°C, annealing for 30 s at 52°C, and extension for 100 s at 72°C (30 circles), and overextension for 10 min at 72°C.

The DNA fragment causes a change of guanine (G) at position 597 in the BBD29_00405 gene coding zone of the mutagenized strain of ATCC13869, *Corynebacterium glutamicum* YPGLU001 (Accession Number of the Biological Deposit: CGMCC No. 21220), finally resulting in a change of the amino acid at position 199 of a coded protein from methionine (M) to isoleucine (I).

After pK18mobsacB plasmid (purchased from Addgene Coporation) is cleaved with *Xbα* I, the BBD29_00405^{G597A}-Up-Down and the linearized pK18mobsacB plasmid are isolated via Agarose Gel Electrophoresis and purified, followed by assembly with NEBuider recombination system (NEB E5520S) to provide vector pK18-BBD29_00405^{G597A}, a plasmid containing a kanamycin-resistant marker. And, the vector pK18-BBD29_00405^{G597A} is sent for sequencing and identification in a company for sequencing, while vector pK18-BBD29_00405^{G597A} containing a correct point mutation (G-A) is preserved for reserve.

Recombinant vector pK18-BBD29_00405^{G597A} is a recombinant vector obtained by inserting the DNA fragment BBD29_00405^{G597A}-Up-Down fragment set forth in SEQ ID NO: 31 between *Xba*I recognition sites of pK18mobsacB vector, while keeping the other sequences of pK18mobsacB vector unchanged.

### Example 2. Construction of Engineered Strain Comprising BBD29_00405 ^{G597A} with Point Mutation

Method for Construction: plasmid pK18-BBD29_00405^{G597A} following allelic replacement is electrotransformed into *Corynebacterium glutamicum* YPGLU001 producing L-glutamic acid in high yield (Accession Number of the Biological Deposit: CGMCC No. 21220; it is confirmed by sequencing that a wild-type BBD29_00405 gene coding zone is retained on the chromosome of the strain); the cultured mono-colonies are each identified with primer P1 and universal primer M13R for a positive strain with a band in about 1554 bp (SEQ ID NO: 32) following amplification. The positive strain is cultured on a culture medium containing 15% sucrose; and the cultured mono-colonies are cultured on culture media with and without kanamycin, respectively. Strains that grow on a culture medium without kanamycin and do not grow on a culture medium with kanamycin are subjected to further identification by PCR with the primers as follows (synthesized by Invitrogen Corporation, Shanghai).
P5: 5'AGAAGGCAACCTGCGCATGA 3' (SEQ ID NO: 9)
P6: 5'ATCGGGTTGGAAATCGCAGA 3' (SEQ ID NO: 10);

The sequence of the universal primer M13R is follows: M13R: 5'CAG GAA ACA GCT ATG ACC3'.

The above amplified product in 261bp (SEQ ID NO: 33) from PCR are subjected to denaturation at a high temperature and ice bath before sscp electrophoresis (with an amplified fragment of plasmid pK18-BBD29_00405^{G597A} as a positive control, an amplified fragment of ATCC13869 as a negative control, and water as a blank control). Since fragments are different in their structures, their locations in electrophoresis are different. Thus, a strain having successful allelic replacement is such a strain that the location of its fragment in electrophoresis is inconsistent with that of a negative control fragment and consistent with that of a positive control fragment.

Again, primers P5 and P6 are used for PCR amplification of a target fragment in a strain having successful allelic replacement, which is linked to PMD19-T vector for sequencing. Base sequence alignment is employed to identify a successful or failed replacement; and the mutant strain derived from a successful replacement of *Corynebacterium glutamicum* YPGLU001 producing glutamic acid in high yield (Accession Number of the Biological Deposit: CGMCC No. 21220) is designated as YPG-025.

The recombinant bacterium YPG-025 is a genetically engineered bacterium YPG-025 containing a point mutation (G-A), as obtained by introducing a point mutation G597A to the BBD29_00405 gene coding zone (SEQ ID NO: 1) of *Corynebacterium glutamicum* CGMCC No. 21220 by virtue of allelic replacement, causing a mutation to the position 597 of the gene from G to A, while keeping the other sequences of the gene unchanged.

Compared with *Corynebacterium glutamicum* CGMCC21220, *Corynebacterium glutamicum* YPG-025 differs only in the substitution of the BBD29_00405 gene set forth in SEQ ID NO: 1 in the genome of *Corynebacterium glutamicum* CGMCC21220 with the BBD29_00405^{G597A} gene set forth in SEQ ID NO: 2. SEQ ID NO: 1 and SEQ ID NO: 2 only differ in one nucleotide, at position 597.

Preparation of PAGE and conditions for SSCP electrophoresis are shown in Table 2 below:

**Table 2 Preparation of PAGE for sscp Electrophoresis**

| Ingredients | Concentration at 8% |
|---|---|
| 40% Acrylamide | 8 mL |
| ddH₂O | 26 mL |
| Glycerol | 4 mL |
| 10 * TBE | 2 mL |
| TEMED | 40 µL, |
| 10% AP | 600 µL |
| Conditions for Electrophoresis | Electrophoresis Tank Placed into Ice, 1 × TBE Buffer, Voltage 120 V for 10 h |

### Example 3. Construction of Engineered Strain with BBD29_00405 or BBD29_00405^{G597A} Gene Overexpressing on Genome

According to the *Corynebacterium glutamicum* ATCC13869 genome (GenBank: CP016335.1) sequence published on NCBI, three pairs of primers are designed and synthesized for amplification of upstream and downstream homology arm fragments, and a sequence of a BBD29_00405 gene coding region and a promoter region, so as to introduce BBD29_00405 or BBD29_00405^{G597A} gene into the strain *Corynebacterium glutamicum* YPGLU001 (Accession Number of the Biological Deposit: CGMCC No. 21220) through homologous recombination.

The primers are designed as follows (synthesized by Invitrogen Corporation, Shanghai):
P7: 5'CAGTGCCAAGCTTGCATGCCTGCAGGTCGACTCTAGGACCCGCTTGCCAT ACGAAG 3'
P8: 5'CCTACCACGA CGAGCACTAC ATCTACTCAT CTGAAGAATC 3'
P9: 5'GATTCTTCAG ATGAGTAGAT GTAGTGCTCG TCGTGGTAGG 3'
P10: 5'CAAACCAGAG TGCCCACGAA CTAGCCGGCG TAAGGATCCC 3'
P11: 5'GGGATCCTTA CGCCGGCTAG TTCGTGGGCA CTCTGGTTTG 3'
P12: 5'CAGCTATGACCATGATTACGAATTCGAGCTCGGTACCCCATAAGAAACAACCACTTCC 3'

Method for Construction: using *Corynebacterium glutamicum* ATCC13869 or YPI019 as a template, PCR amplification is performed with primers P7/P8, P9/P10, and P11/P22, respectively, to provide an upstream homologous arm fragment in about 806bp, a fragment of BBD29_00405 (SEQ ID NO: 34) or BBD29_00405^{G597A} (SEQ ID NO: 36) gene in about 1777bp, and a downstream homologous arm fragment in about 788bp (SEQ ID NO: 37). Further, using P7/P12 as primers, amplification is performed with a mixture of the three amplified fragments above as a template to provide an integrated homologous arm fragment upstream-BBD29_00405-downstream (SEQ ID NO: 38) or an integrated homologous arm fragment upstream-BBD29_00405^{G597A}-downstream (SEQ ID NO: 39) in 3291bp. After the PCR reaction is completed, electrophoresis is performed on the amplified products for recovering a desired DNA fragment in about 3291bp using a Column DNA Gel Recovery Kit, which is ligated, using NEBuider recombination system, to shuttle plasmid PK18mobsacB having been cleaved with *Xbα* I and recovered to provide an integrative plasmid (i.e., a recombinant vector) PK18mobsacB-BBD29_00405 or PK18mobsacB-BBD29_00405^{G597A}. The plasmid contains a kanamycin-resistant marker through which a recombinant having a plasmid integrated into genome can be obtained by screening with kanamycin.

pk18mobsacB-BBD29_00405 is a recombinant vector obtained by inserting the integrated homologous arm fragment upstream-BBD29_00405-downstream (SEQ ID NO: 38) between the cleavage sites of *Xba* I of shuttle plasmid pk18mobsacB.

pk18mobsacB-BBD29_00405 is a recombinant vector obtained by inserting the integrated homologous arm fragment upstream-BBD29_00405^{G597A}-downstream (SEQ ID NO: 39) between the cleavage sites of *Xba* I of shuttle plasmid pk18mobsacB.

PCR System: 10×Ex Taq Buffer 5 µL, dNTP Mixture (2.5 mM each) 4 µL, Mg²⁺(25 mM) 4 µL, primers (10 pM) 2 µL each, Ex Taq (5 U/µL) 0.25 µL, a template 1 µL, with water as balance, in a total volume of 50 µL.

The above PCR amplification is performed by pre-denaturation for 5 min at 94°C, denaturation for 30 s at 94°C, annealing for 30 s at 52°C, and extension for 180 s at 72°C (30 circles), and overextension for 10 min at 72°C.

The two integrative plasmids are each electrotransformed into *Corynebacterium glutamicum* YPGLU001 (Accession Number of the Biological Deposit: CGMCC No. 21220), and PCR identification is performed on the cultured mono-colonies with primers P13/P14 for a positive strain containing a fragment in about 1970bp (SEQ ID NO: 40) from PCR amplification, while a strain without any fragments amplified therefrom is an original one. Having being screened with 15% sucrose, the positive strain is cultured on culture media with and without kanamycin, respectively, and strains that grow on a culture medium without kanamycin and do not grow on a culture medium with kanamycin are further subjected to PCR for identification with primers P15/P16. The bacterium with a fragment amplified therefrom in about 1758bp (SEQ ID NO: 41) is a strain having BBD29_00405 or BBD29_00405^{G597A} gene integrated into the genome of the strain *Corynebacterium glutamicum* YPGLU001 (Accession Number of the Biological Deposit: CGMCC No. 21220), designated as YPG-026 (without a mutation point) and YPG-027 (with a mutation point).
P13: 5' GTCCAAGGTGACGGCCGCAC 3'
P14: 5' AGCTTCGCCGATGTTGCGCA 3'
P15: 5'AGGTTGCACCCGCCATCGCTGCA3'
P16: 5' ATATTCGGCCCAGCAGCAGC 3'

The recombinant bacterium YPG-026 is a recombinant bacterium containing a double-copy BBD29_00405 gene set forth in SEQ ID NO: 1, as obtained by integrating the integrated homologous arm fragment upstream-BBD29_00405-downstream (SEQ ID NO: 38) to the genome of the strain *Corynebacterium glutamicum* YPGLU001. The recombinant bacterium containing a double-copy BBD29_00405 gene can significantly and stably increase the expression amount of the BBD29_00405 gene.

The recombinant bacterium YPG027 is a recombinant bacterium containing a mutated BBD29_00405^{G597A} gene set forth in SEQ ID NO: 2, as obtained by integrating the integrated homologous arm fragment upstream-BBD29_00405^{G597A}-dowmstream (SEQ ID NO: 39) to the genome of the strain *Corynebacterium glutamicum* YPGLU001.

### Example 4. Construction of Engineered Strain Overexpressing BBD29_00405 or BBD29_00405^{G597A} Gene on Plasmid

According to the *Corynebacterium glutamicum* ATCC13869 genome (GenBank: CP016335.1) sequence published on NCBI, a pair of primers for amplifying a sequence of a BBD29_00405 gene coding region and a promoter region are designed and synthesized. The primers are designed as follows (synthesized by Invitrogen Corporation, Shanghai).
P17: 5' GCTTGCATGCCTGCAGGTCGACTCTAGAGGATCCCCGTAGTGCTCGTCGTGGTAGG 3'
P18: 5' ATCAGGCTGAAAATCTTCTCTCATCCGCCAAAACCTAGCCGGCGTAAGGATCCCGGAT 3'

Method for Construction: using ATCC13869 or YPG-025 as a template, PCR amplification is performed with primers P17/P18 to provide a DNA molecule containing BBD29_00405 (SEQ ID NO: 42) or a DNA molecule containing BBD29_00405^{G597A} (SEQ ID NO: 43) in about 1807bp. Electrophoresis is performed on the amplified products for recovering a desired DNA fragment in 1807 bp using a Column DNA Gel Recovery Kit, which is ligated, using NEBuider recombination system, to shuttle plasmid pXMJ19 (BioVector NTCC BiovectorpXMJ19) having been cleaved with *EcoR* I and recovered to provide an overexpression plasmid pXMJ19-BBD29_00405 or pXMJ19-BBD29_00405^{G597A}. The plasmid contains a chloramphenicol-resistant marker, and the transformation of the plasmid into the strain can be achieved by screening with chloramphenicol.

PCR System: 10 × Ex Taq Buffer 5 µL, dNTP Mixture (2.5 mM each) 4 µL, Mg²⁺ (25 mM) 4 µL, primers (10 pM) 2 µL each, Ex Taq (5 U/µL) 0.25 µL, a template 1 µL, with water as balance, in a total volume of 50 µL.

The above PCR amplification is performed by pre-denaturation for 5 min at 94°C, denaturation for 30 s at 94°C, annealing for 30 s at 52°C, and extension for 100 s at 72°C (30 circles), and overextension for 10 min at 72°C.

The recombinant vector pXMJ19-BBD29_00405 is a recombinant strain obtained by inserting the DNA molecule containing BBD29_00405 (SEQ ID NO: 42) between the cleavage sites of *EcoR* I of shuttle plasmid pXMJ19.

The recombinant vector pXMJ19-BBD29_00405^{G597A} is a recombinant strain obtained by inserting the DNA molecule containing BBD29_00405^{G597A}(SEQ ID NO: 43) between the cleavage sites of *EcoR* I of shuttle plasmid pXMJ19.

Plasmids are each electrotransformed into *Corynebacterium glutamicum* YPGLU001 (Accession Number of the Biological Deposit: CGMCC No. 21220), and PCR identification is performed on the cultured mono-colonies with primers M13R (-48) and P18 for a transformed strain containing a fragment in about 1846bp (SEQ ID NO: 44) from PCR amplification, designated as YPG-028 (without a mutation point) and YPG-029 (with a mutation point).

The sequence of M13R (-48) is as follows:
5'AGCGGATAAC AATTTCACAC AGGA3'

The recombinant bacterium YPG-028 contains a double-copy BBD29_00405 gene set forth in SEQ ID NO: 1; the recombinant bacterium YPG-028 is an engineered bacterium overexpressing a wild-type BBD29_00405 gene on a plasmid, i.e., overexpression outside a chromosome via plasmid pXMJ19-BBD29_00405.

The recombinant bacterium YPG-029 contains a mutated BBD29_00405^{G597A} gene set forth in SEQ ID NO: 2; the recombinant bacterium YPG-029 is an engineered bacterium overexpressing a mutant-type BBD29_00405^{G597A} gene on a plasmid, i.e., overexpression outside a chromosome via plasmid pXMJ19-BBD29_00405^{G597A};

### Example 5. Construction of Engineered Strain with BBD29_00405 Gene Deleted on Genome

According to the *Corynebacterium glutamicum* ATCC13869 genome (GenBank: CP016335.1) sequence published on NCBI, two pairs of primers for amplifying fragments at both ends of a BBD29_00405 gene coding region are synthesized, as upstream and downstream homologous arm fragments. The primers are designed as follows (synthesized by Invitrogen Corporation, Shanghai):
P19: 5'CAGTGCCAAGCTTGCATGCCTGCAGGTCGACTCTAGGTCTGGGGGTGAGCGCGGAT 3'
P20: AGGAAAATAACGCATCCATCTGCCCCTTTACAAATCCACCGCAAACACTGGGAT 3'
P21: TGGATTTGTAAAGGGGCAGATGGATGCGTTATTTTCCTTCACTTTTCGTATCCA 3'
P22: 5'CAGCTATGACCATGATTACGAATTCGAGCTCGGTACCCCTCTGGCGCATCGAACAGGT CGAAGGA 3'

Using *Corynebacterium glutamicum* ATCC13869 as a template, PCR amplification is performed with primers P19/P20 and P21/P22, respectively, to provide an upstream homologous arm fragment in 709bp (SEQ ID NO: 45) and a downstream homologous arm fragment in 734bp (SEQ ID NO: 46). Further, primers P19/P22 are used for an OVER PCR to provide an integral homologous arm fragment in 1405bp (SEQ ID NO: 47). After the PCR reaction is completed, electrophoresis is performed on the amplified products for recovering a desired DNA fragment in 1405bp using a Column DNA Gel Recovery Kit, which is ligated, by virtue of NEBuider recombination system, to a shuttle plasmid, plasmid pk18mobsacB, having been cleaved with *Xbα* I and recovered to provide a knockout plasmid. The plasmid contains a kanamycin-resistant marker.

The knockout plasmid is electrotransformed into *Corynebacterium glutamicum* YPGLU001 (Accession Number of the Biological Deposit: CGMCC No. 21220), and PCR identification is performed on the cultured mono-colonies each with the following primers (synthesized by Invitrogen Corporation, Shanghai):
P23: 5'GTCTGGGGGTGAGCGCGGAT3'
P24: 5'CTCTGGCGCATCGAACAGGTCGAAGGA 3'

A strain with bands in about 1331bp (SEQ ID NO: 49) and about 2804bp (SEQ ID NO: 48) amplified from the above PCR amplification is a positive one, while a strain with just a band in 2804bp amplified therefrom is a starting one. Having being screened on a culture medium with 15% sucrose, the positive strain is cultured on culture media with and without kanamycin, respectively, and strains that grow on a culture medium without kanamycin and do not grow on a culture medium with kanamycin are subjected further to PCR identification with primers P23/P24. The strain with a band in 1331bp amplified therefrom is a genetically engineered strain with the BBD29_00405 gene coding region knocked out, designated as YPG-030.

The recombinant bacterium YPG-030 is one having the BBD29_00405 gene on the genome of *Corynebacterium glutamicum* CGMCC No. 21220 knocked out.

### Example 6. Experiments on Fermentation of L-glutamic acid

Experiments on fermentation are performed on the strains YPG-025, YPG-026, YPG-027, YPG-028, YPG-029 and YPG-030 as constructed in Examples 2-5, and an original *Corynebacterium glutamicum* YPGLU001 (Accession Number of the Biological Deposit: CGMCC No. 21220) in a fermentation tank, BLBIO-5GC-4-H type (purchased from Shanghai Bailun Biological Technology Co., Ltd.) with the culturing medium shown in Table 3 and the control process for fermentation shown in Table 4, and products from fermentation are collected.

At the initial moment when seeding is completed, the bacterium is at a concentration of 15 g/L in the system. During the fermentation: sugar content in the system (residual sugar) is controlled by supplementing an aqueous solution containing 50-55% glucose.

Triplicates are made for each strain. Results are shown in Table 5.

**Table 3 Formulation of Solutes in Culture Medium for Fermentation**

| Name of Reagent | Compounding Ratio |
|---|---|
| Glucose | 5.0 g/L |
| Phosphoric Acid | 0.38 g/L |
| Magnesium Sulfate | 1.85 g/L |
| Potassium Chloride | 1.6 g/L |
| Biotin | 550 µg/L |
| Vitamin B1 | 300 µg/L |
| Ferrous Sulfate | 10 mg/L |
| Manganese Sulfate | 10 g/dL |
| KH₂PO₄ | 2.8 g/L |
| Vitamin C | 0.75 mg/L |
| Vitamin B12 | 2.5 µg/L |
| Para-Aminobenzoic acid | 0.75 mg/L |
| Defoamer | 0.0015 mL/dL |
| Betaine | 1.5 g/L |
| Cane-Sugar Molasses | 7 mL/L |
| Corn Steep Liquor | 77 mL/L |
| Aspartic acid | 1.7 g/L |
| Hair powder | 2 g/L |

The above culturing medium for fermentation is one obtained by dissolving the solutes shown in Table 3 in water.

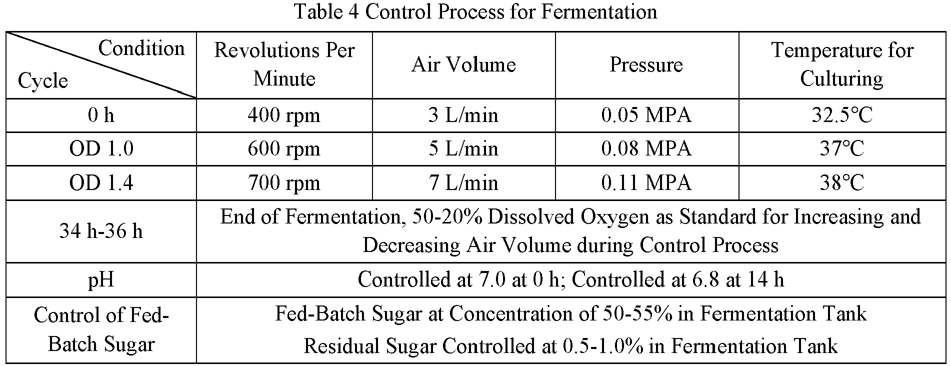

**Table 5 Results of Experiments on Fermentation of L-glutamic Acid**

| Strain | Production of L-Glutamic Acid (g/L) | OD (562 nm) |
|---|---|---|
| *Corynebacterium glutamicum* YPGLU001 | 97.6 | 41.5 |
| YPG-025 | 96.1 | 40.3 |
| YPG-026 | 98.3 | 42.9 |
| YPG-027 | 101.5 | 41.6 |
| YPG-028 | 99.7 | 41.1 |
| YPG-029 | 102.9 | 40.8 |
| YPG-030 | 89.4 | 41.4 |

Results from Table 5 show that, in an engineered bacterium producing L-glutamic acid, *Corynebacterium glutamicum* YPGLU001 (Accession Number of the Biological Deposit: CGMCC No. 21220), point mutation to the BBD29_00405 gene coding region, BBD29_00405^{G597A} facilitates an increase in the production of L-glutamic acid; overexpression of BBD29_00405 or BBD29_00405^{G597A} facilitates to increase the production of L-glutamic acid, while knocking out the BBD29_00405 gene is adverse to the accumulation of L-glutamic acid.

The embodiments of the present invention have been illustrated above. However, the present invention is not limited to the above embodiments. Any modification, equivalent substitution, improvement, etc. made within the spirit and principle of the invention should be included in the scope of protection of the present invention.

### Industrial Application

The present invention has found out that, by knocking out the BBD29_00405 gene, the product coded by the gene has an effect on L-glutamic acid production capacity, and that a recombinant strain obtained by introducing a point mutation to the coding sequence, or by increasing the copy number of, or overexpressing the gene facilitates production of L-glutamic acid at a higher concentration as compared with an unmodified strain. When the inhibitor of CTD-2256P15.2 or the micropeptide PACMP coded by it provided by the present invention acts on tumor cells or tumor tissues, the growth of tumor cells can be remarkably inhibited, the apoptosis of tumor cells can be increased, the tumor volume can be reduced, and an excellent anti-tumor effect can be achieved. The novel anti-tumor drugs combination scheme provided by the present invention, use of the inhibitor of CTD-2256P15.2 or the micropeptide PACMP coded by it in combination with other anti-tumor drugs, can significantly enhance the killing effect of the anti-tumor drugs on tumor cells and reduce the resistance of tumor cells to chemotherapy, thereby improving the clinical treatment effect on tumors. CTD-2256P15.2 is highly expressed in chemotherapy-resistant tumor tissues and cell lines, and its high expression is significantly negatively correlated with progression-free survival and overall survival of patients with tumors. The CTD2256P15.2 gene expression level provided by the present invention can be used as a molecular index for predicting the sensitivity to chemotherapy and prognosis of patients with tumors, creating a new standard for an effective guide to clinical chemotherapeutic medication for patients with tumors and evaluating the prognosis of treatment.

Specifically, the present invention first constructs a genetically engineered bacterium YPG-025 with a point mutation **(G-A)** by introducing a point mutation to a BBD29_00405 gene coding region (SEQ ID NO: 1) of *Corynebacterium glutamicum* CGMCC No. 21220 via allelic replacement. In order to further investigate and verify that overexpressing a wild-type BBD29_00405 gene or a mutant gene BBD29_00405^{G597A} thereof in a producer bacterium can increase the production of L-glutamic acid, an exogenous gene is integrated into the chromosome of a host, or expressed outside the chromosome by a plasmid, respectively, thereby constructing engineered bacteria YPG-026, YPG-027, YPG-028 and YPG-029 overexpressing BBD29_00405 gene or BBD29_00405^{G597A} gene on genome and plasmid. Experiments suggest that BBD29_00405 gene and variants thereof are involved in the biosynthesis of L-glutamic acid. By overexpressing or knocking out BBD29_00405 gene, or having site-directed mutation (such as point mutation) thereto, the amount of accumulation of L-glutamic acid in a microorganism can be regulated. Point mutation to the BBD29_00405 gene coding region or overexpression of BBD29_00405 gene or a mutant gene BBD29_00405^{G597A} thereof in a producer bacterium facilitates an increase in the production and conversion rate of L-glutamic acid, while knocking out or weakening the BBD29_00405 gene is adverse to the accumulation of L-glutamic acid. BBD29_00405 gene and a variant thereof (such as BBD29_00405^{G597A} gene) can be used to construct a genetically engineered strain for producing L-glutamic acid to promote an increase in the production of L-glutamic acid, and to breed a high-production and high-quality strain for industrialized production, which finds values in a wide range of applications to, and is of important economic significance for industrialized production of L-glutamic acid.

## Claims

1. A bacterium for generating L-glutamic acid, **characterized in** having an improved expression of a polynucleotide encoding an amino acid sequence of SEQ ID NO: 3;
preferably, the improved expression is an enhanced expression of the polynucleotide encoding an amino acid sequence of SEQ ID NO: 3, or having a point mutation in the polynucleotide encoding an amino acid sequence of SEQ ID NO: 3, or having a point mutation in, and an enhanced expression of the polynucleotide encoding an amino acid sequence of SEQ ID NO: 3.

2. The bacterium of claim 1, **characterized in** a point mutation to the polynucleotide encoding an amino acid sequence of SEQ ID NO: 3 such that methionine at position 199 in the amino acid sequence of SEQ ID NO: 3 is substituted with a different amino acid; preferably, methionine at position 199 is substituted with isoleucine.

3. The bacterium of claim 1 or 2, **characterized in that** the polynucleotide encoding an amino acid sequence of SEQ ID NO: 3 comprises a nucleotide sequence of SEQ ID NO: 1.

4. The bacterium of any one of claims 1-3, **characterized in that** the polynucleotide sequence having a point mutation is formed from a mutation to the base at position 597 of a polynucleotide sequence set forth in SEQ ID NO: 1;
preferably, the mutation comprises a mutation of the base at position 597 of a polynucleotide sequence set forth in SEQ ID NO: 1 from guanine (G) to adenine (A);
preferably, the polynucleotide sequence having a point mutation comprises a polynucleotide sequence set forth in SEQ ID NO: 2.

5. The bacterium of any one of claims 1-4, **characterized in that** the bacterium is a bacterium of the genus *Corynebacterium,* preferably, *Corynebacterium acetoacidophilum, Corynebacterium acetoglutamicum, Corynebacterium callunae, Corynebacterium glutamicum, Brevibacterium flavum, Brevibacterium lactofermentum, Corynebacterium ammoniagenes, Corynebacterium pekinense, Brevibacterium saccharolyticum, Brevibacterium roseum,* and *Brevibacterium thiogenitalis*; preferably *Corynebacterium glutamicum* YPGLU001, Accession Number of Biological Deposit: CGMCC No. 21220, or *Corynebacterium glutamicum* ATCC 13869.

6. A method for producing L-glutamic acid, the method comprising: culturing the bacterium of any one of claims 1-5 and recovering L-glutamic acid from the culture.

7. A polynucleotide, **characterized in** comprising a polynucleotide encoding containing an amino acid sequence set forth in SEQ ID NO: 3, wherein methionine at position 199 is substituted with a different amino acid; preferably, methionine at position 199 is substituted with isoleucine;
preferably, the polynucleotide comprises a polynucleotide encoding containing an amino acid sequence set forth in SEQ ID NO: 4;
preferably, the polynucleotide is formed from a mutation to the base at position 597 of a polynucleotide sequence set forth in SEQ ID NO: 1; preferably, the mutation is a mutation of the base at position 597 of the polynucleotide sequence set forth in SEQ ID NO: 1 from guanine (G) to adenine (A);
preferably, the polynucleotide comprises a polynucleotide sequence set forth in SEQ ID NO: 2.

8. A protein, **characterized in that** the amino acid sequence of the protein is set forth in SEQ ID NO: 4.

9. A recombinant vector, an expression cassette, a transgenic cell line and/or recombinant bacterium comprising the polynucleotide of claim 7 and/or the protein of claim 8.

10. Use of the polynucleotide of claim 7, the protein of claim 8, the recombinant vector, the expression cassette, the transgenic cell line and/or recombinant bacterium of claim 9 in the production of L-glutamic acid.

11. A protein, **characterized in that** the protein is any one of:
A1) a protein whose amino acid sequence is SEQ ID NO: 4;
A2) a protein having 80% or more identity to and the same function as the protein indicated in A1), as obtained by subjecting the amino acid sequence set forth in SEQ ID NO: 4 to substitution and/or deletion and/or addition of amino acid residues;
A3) a fusion protein having the same function, as obtained by linking a tag to the N-terminus and/or C-terminus of A1) or A2).

12. A nucleic molecule, **characterized in that** the nucleic acid molecule is any one of:
B1) a nucleic acid molecule encoding the protein of claim 11;
B2) a DNA molecule whose coding sequence is set forth in SEQ ID NO: 2.
B3) a DNA molecule whose nucleotide sequence is set forth in SEQ ID NO: 2.

13. A biomaterial, **characterized in that** the biomaterial is any one of:
C1) an expression cassette comprising the nucleic acid molecule of claim 12;
C2) a recombinant vector comprising the nucleic acid molecule of claim 12, or a recombinant vector comprising the expression cassette of C1);
C3) a recombinant microorganism comprising the nucleic acid molecule of claim 12, or a recombinant microorganism comprising the expression cassette of C1), or a recombinant microorganism comprising the recombinant vector of C2).

14. Any one of the following uses of any one of D1)-D8):
F1) use of any one of D1)-D8) in the regulation of the production of L-glutamic acid of a microorganism;
F2) use of any one of D1)-D8) in the construction of a genetically engineered bacterium for producing L-glutamic acid;
F3) use of any one of D1)-D8) in the preparation of L-glutamic acid;
wherein the D1)-D8) is:
D1) the protein of claim 11;
D2) the nucleic acid molecule of claim 12;
D3) the biomaterial of claim 13;
D4) a DNA molecule whose nucleotide sequence is SEQ ID NO: 1;
D5) a DNA molecule having 90% or more identity to and the same function as the DNA molecule set forth in SEQ ID NO: 1, as obtained by subjecting the nucleotide sequence set forth in SEQ ID NO: 1 to modification, and/or substitution and/or deletion and/or addition of one or several nucleotides;
D6) an expression cassette comprising the DNA molecule in D4) or D5);
D7) a recombinant vector comprising the DNA molecule in D4) or D5), or a recombinant vector comprising the expression cassette of D6);
D8) a recombinant microorganism comprising the DNA molecule in D4) or D5), or a recombinant microorganism comprising the expression cassette of D6), or a recombinant microorganism comprising the recombinant vector of D7).

15. A method for increasing the production of L-glutamic acid in a microorganism, **characterized in that** the method comprises any one of:
E1) increasing the expression amount, or content of the nucleic acid molecule of claim 12 in a target microorganism to provide a microorganism having a greater production of L-glutamic acid than the target microorganism;
E2) increasing the expression amount, or content of the DNA molecule of D4) or D5) in claim 14 in a target microorganism to provide a microorganism having a greater production of L-glutamic acid than the target microorganism;
E3) performing a mutation on the DNA molecule whose nucleotide sequence is SEQ ID NO: 1 in the target microorganism to provide a microorganism having a greater production of L-glutamic acid than the target microorganism.

16. The method of claim 15, **characterized in that** the mutation is a point mutation.

17. The method of claim 16, **characterized in that** the point mutation is a mutation of methionine residue at position 199 in an amino acid sequence coded by the DNA molecule set forth in SEQ ID NO: 1 to another amino acid residue.

18. The method of claim 16 or 17, **characterized in that** the point mutation is a mutation of alanine at position 199 in an amino acid sequence coded by the DNA molecule set forth in SEQ ID NO: 1 to isoleucine, providing a mutated protein whose amino acid sequence is SEQ ID NO: 4.

19. A method for constructing the recombinant microorganism of claim 13 or 14, **characterized in that** the method comprises at least one of:
F1) introducing the nucleic acid molecule of claim 12 into a target microorganism to provide the recombinant microorganism;
F2) introducing the DNA molecule set forth in SEQ ID NO: 1 into a target microorganism to provide the recombinant microorganism;
F3) editing the DNA molecule set forth in SEQ ID NO: 1 with a gene editing measure to contain the DNA molecule set forth in SEQ ID NO: 2 in a target microorganism.

20. A method for preparing L-glutamic acid, **characterized in that** the method comprises producing L-glutamic acid with the recombinant microorganism of claim 13 or 14.
